# EUROPÄISCHE PATENTSCHRIFT

(11) **EP 1 606 622 B1**
(45) Veröffentlichungstag und Bekanntmachung des Hinweises auf die Patenterteilung: **01.09.2010**
(21) Anmeldenummer: 04718197.9
(22) Anmeldetag: 06.03.2004
(51) Int. Cl.: G01N 33/573, C12Q 1/44, C12Q 1/26, C12Q 1/527

(54) **TESTMETHODEN ZUR BESTIMMUNG DER INTRAZELLULÄREN KONZENTRATION ZYKLISCHER NUKLEOTIDE**
TEST METHODS FOR DETERMINING THE INTRACELLULAR CONCENTRATION OF CYCLIC NUCLEOTIDES
PROCEDES D'ESSAI PERMETTANT DE DETERMINER LA CONCENTRATION INTRACELLULAIRE DES NUCLEOTIDES CYCLIQUES

(30) Priorität: 18.03.2003 DE 10311769
(43) Veröffentlichungstag der Anmeldung: 21.12.2005
(73) Patentinhaber: AXXAM S.p.A., 20132 Milano (IT)
(72) Erfinder: WUNDER, Frank, 42117 Wuppertal (DE)
(74) Vertreter: Banfi, Paolo
(86) Internationale Anmeldenummer: PCT/EP2004/002317
(87) Internationale Veröffentlichungsnummer: WO 2004/083803

(56) Entgegenhaltungen:
- DE-A- 10 138 876
- SAUTTER A ET AL: "An isoform of the rod photoreceptor cyclic nucleotide-gated channel beta subunit expressed in olfactory neurons." PROCEEDINGS OF THE NATIONAL ACADEMY OF SCIENCES OF THE UNITED STATES OF AMERICA. 14 APR 1998, vol. 95, no. 8, 14 April 1998 (1998-04-14), pages 4696-4701, XP002285826 ISSN: 0027-8424 cited in the application

### SEQUENCE LISTING

<110> Bayer AG, BHC
<120> Testmethoden zur Bestimmung der intrazellulären Konzentration zyklischer Nukleotide
<130> Le A 36 621
<160> 5
<170> PatentIn version 3.1
<210> 1
   <211> 3166
   <212> DNA
   <213> Bos taurus
<400> 1
   gctcacctac tggcaggctg gggtgtgcag gccccacagt gagagggtca gacatccagc 60
   cagtgggcag aactgtttct ccagtgggca agtgctgccc tgggccgagg cctcgacctc 120
   gcctcagctg taggcatggg ctcctctgag tcactgccgg cctcccctgc tggcctcagg 180
   cagggcgggc agctctttga ctgagaggct gagaagctgc gtggggattc ggggatcccc 240
   ctggtctggg gacaggagct ttgggaggtc tctactgtcc cttggtgctg atgagctccc 300
   gagggttcgt ctctgactgg aagcttcttg gacagaccta tagcctgtgg ccaagggaca 360
   ccctgcctcg gaatccgtct ggtgaggaaa ggtgagggtc ctggttgtac atggaggatg 420
   acagaaaaag ccaatggcgt gaagagctcc ccagccaata accacaacca ccatgcccct 480
   cctgccatca aggccagtgg caaagatgac cacagggcca gcagccggcc acagtctgct 540
   gctgctgatg acacctcctc agagctacag caactggcag agatggatgc cccccagcag 600
   aggaggggtg gcttccgcag gattgcccgc ctggtggggg tcctcagaga gtgggcttac 660
   aggaacttcc gtgaggagga gcctagacct gactcattcc ttgagcgttt ccgggggcct 720
   gagctccaca ccgtgacaac acaacaagga gacggcaaag gcgacaagga cggcgagggc 780
   aagggcacca agaagaagtt tgaactcttt gtcttggacc cagccgggga ctggtactac 840
   cgctggcttt ttctcattgc cttgcccgtc ctctacaact ggtgcctatt ggtggccaga 900
   gcctgcttca gtgacctgca gaaaggctac tacatagtgt ggctggtgct ggattacgtc 960
   tcagatgtgg tctacatcgc agacctcttc atccgactgc gcacaggttt cttggagcag 1020
   gggctactgg tgaaagacac caagaagttg cgggacaact acatccacac catgcagttt 1080
   aagctggatg tggcctccat catccctaca gacctgatct attttgctgt ggggatccat 1140
   aaccctgagg tgcgcttcaa ccgcctgcta cactttgccc gcatgtttga gttctttgac 1200
   cgcactgaga cacgcaccag ctaccccaac atcttccgaa taagcaacct gatcctctac 1260
   atcttgatca tcattcactg gaatgcctgc atctactatg ccatctccaa gtccatcggc 1320
   tttggggtag acacctgggt ttaccccaac atcactgacc ctgagtatgg ctacctgtct 1380
   agggagtaca tctattgcct ttactggtct acactgaccc tcaccaccat tggggagaca 1440
   ccaccccctg taaaggatga ggagtacctg tttgtcatct ttgacttcct gattggtgtc 1500
   ctcatctttg ccaccatcgt gggaaatgtg ggctccatga tctccaacat gaatgccacc 1560
   cgggctgagt tccaggccaa gattgatgct gtcaaacatt atatgcagtt ccgaaaggtc 1620
   agcaaggaga tggaagccaa ggtcattagg tggtttgact acttgtggac caataagaag 1680
   agtgtagatg agcgagaagt cctcaaaaac ctgccagcaa agctcagggc tgagatagcc 1740
   atcaacgtcc acctgtccac actcaagaaa gtgcgcatct ttcaggactg tgaggctggc 1800
   ctgctggtgg aactggtatt aaagctccgg cctcaggtct ttagccctgg ggactacatt 1860
   tgccgcaagg gggatattgg gaaggagatg tacataatca aggagggaaa attggcagtg 1920
   gtggctgatg acggtgtcac tcagtatgcc ctgctctcgg ctgggagttg ctttggagag 1980
   atcagtatcc ttaatattaa gggcagcaaa atgggcaatc ggcgcacagc caacatccgc 2040
   agtcttggct actctgatct gttctgcttg tccaaggatg atcttatgga agctgtgact 2100
   gagtaccctg atgccaagag ggtcttggag gagagaggcc gggagattct gatgaaggag 2160
   ggcttgttgg atgagaatga ggtggcagcc agcatggagg tagatgtgca ggaaaagcta 2220
   gaacagctgg agaccaacat ggacaccttg tacactcgtt ttgcccgcct gctggccgag 2280
   tacacgggag cccagcagaa gctcaagcag cgcatcacag ttttggaaac gaagatgaag 2340
   cagaataatg aggatgactc cctgtcagat gggatgaaca gcccagagcc acctgccgag 2400
   aagccataat ggcttggccc aattgcccct ccagccttgg ctttgacccc agggctggaa 2460
   gagctgtgta ggtccccaca tatatatgca ttaccacatc cccttgaatt ctcccagaag 2520
   cctctctgct ggaaggttta gggctcgatc atccagaagc cctcctccaa gtccgactaa 2580
   cagctaatct tgtgcagggc atagactgtg cttagctcgg cttccagaag cttcagcctg 2640
   tctaagtttg aggaagaaag aaaagaggag catctctcca ggctcttttg catctagtta 2700
   cctcctactt gattcttttc taatatgtgt tctgaatatt tccatttccc tgcagcagta 2760
   tgtagttaga acactggctg cagacaccca gcactggtcc cagtgtcctt ttcccaaggc 2820
   aggcaaaagg tgtggagggg ggcaaggagg agatgctcac tccaagtcct gccgtgctga 2880
   ttcctccgcc tgtctgccaa cccagagtgg gagccctgtg gtcttttctg aaccaggggg 2940
   aggaggatgc tcctggtctc caatccatcc caggacatgg agtgaggaac tagcagttgg 3000
   ccagcaggca aggcacctgg agaaggtggt gggcaggagc ctggccatca cccctctatg 3060
   cagagtgttt ctcaggaggc cctgaggctg atggtgggtg gatgactctt caagttaaca 3120
   tctgcagtag agacacttac aagttaataa attcctctga actttt 3166
<210> 2
   <211> 3236
   <212> DNA
   <213> Rattus norvegicus
<400> 2
   caccagctcc atgctgtgcc aatcacagcg tcttagaagc tgctgtcctg gctgacccag      60
   ggcttctccc ggctccactc atatgctcca gtcccaggct tggcatcact gtcacagccc     120
   caggctgacc tactgcaaca tggaccttcc taccagcaga gcccgaaaga cccaatgtcc     180
   tgctggatag ctgtttggtg gtgcaggctg atgtggacca gtgccagcta gaaagggcac     240
   agccagagac agcatcgatc caggagttac cagaagaaga agaggagaag gaggaagaga     300
   agaaggagga ggaagaggag aaggaggagg aggaagagaa ggaagaggaa gaggagaagg     360
   aggaggaggg ggaggccaca aactcaacag tccccaggat cacgcctctg cctgacacct     420
   acgggaccca gtaccatggc taaaccaggc ctgaacatgg agctgaatcg cctggtccag     480
   gaccagccac ctggccagga gggccccaga cctggccccc caaacccagc cgagcacctc     540
   cccaatgtgc ccagctaccg tcccgcaacc acccgcatcc ccgtcctcgt ctcccggagg     600
   acagccttgt ccaactccaa cttcaccaag gagatcagga gctccatccg tcgcctggta     660
   ccagccacga aagagcaccc ggagctccag gtggaagaca cagatgccga agctggcccc     720
   ctcatcccag aggagacgat cccgccacct gagagaccac cagtgtctcc cgccaagtct     780
   gacaccctcg cggttcccag cgcagcaacc cacaggaaga agctaccttc tcaggatgat     840
   gaggctgaag aactcaaggc cctgtcaccg gctgagtccc cagtggttgc ctggtcagac     900
   cccaccaccc cacaggaggc tgatggcgag gaccgtgcgg cctccacagc cagccagaac     960
   agtgccatca tcaacgaccg gctccaggag ctggtgaaga tgttcaagga gcggacagag    1020
   aaggtgaagg agaagctcat tgaccctgac gtcacctccg atgaggagag ccccaagccc    1080
   tccccagcca agaaggcccc agactcagcc ccagcccaga agccggcgga ggcagaggcg    1140
   gcagaggagg agcactactg tgacatgctc tgctgcaagt tcaagcgcag gccctggaag    1200
   atgtaccagt tcccccagag catcgaccca ctgaccaacc tcatgtacat cctgtggctg    1260
   ttcttcgtgg tgctggcctg gaactggaac tgctggctga ttcctgtgcg ctgggccttc    1320
   ccgtaccagc gggcagacaa catccacctc tggctgctca tggactactt gtgcgacttc    1380
   atctacctcc tggacatcac cgtgttccag atgcgtctcc agtttgtcaa aggcggggac    1440
   atcattacag ataagaagga gatgcgtaat aattacctga agtctcaacg atttaagatg    1500
   gacttgctct gccttttgcc tttggatttt ctctacttga aacttggcgt gaaccccctt     1560
   cttcgcctgc cccgctgcct gaagtacatg gccttctttg agtttaataa ccgtctggaa    1620
   gccatcctca gcaaagccta cgtttacagg gttatcagga ccaccgccta cctgctgtat    1680
   agcttgcatc tcaactcctg tctttactac tgggcgtcgg ccttccaggg catcggttcc    1740
   actcactggg tttatgacgg cgtggggaac agctacattc gatgctacta ctgggctgtg    1800
   aaaactctca tcaccatcgg aggactgccc gacccccaga cgctctttga gatcgtcttc    1860
   cagctgctga attattttac aggtgtcttc gctttctctg tgatgattgg acagatgaga    1920
   gatgtggtgg gggccgccac ggcagggcag acgtactacc gcagctgcat ggacagcacg    1980
   gtgaagtaca tgaacttcta caagatcccc aggtctgtgc agaaccgcgt caagacctgg    2040
   tatgagtaca cctggcattc acaaggcatg ctggatgagt cagagctgat ggttcagctt    2100
   ccggacaaga tgcgtctgga cctggccatt gacgtaaact acaacattgt cagcaaagtg    2160
   gcgctcttcc agggctgcga ccggcagatg atcttcgaca tgctcaagcg acttcgctca    2220
   gtcgtctacc tacccaatga ctatgtgtgc aagaaggggg agattggccg agagatgtat    2280
   attatccagg cggggcaggt gcaggtgctg ggcggcccag atggaaaggc tgtcctggtg    2340
   acactcaaag ccggatcggt gtttggagag ataagcttgc tggctgtcgg gggcggtaac    2400
   aggcgcacgg ccaatgtggt ggcccacggc ttcaccaatc tcttcattct ggataagaag    2460
   gacttgaatg agattttggt gcattaccct gaatctcaga agctgctccg gaagaaggcc    2520
   aggcgcatgc tcagaaacaa caacaaaccc aaggaggaga agagtgtgct catcctgccc    2580
   ccacgtgcgg gcaccccgaa gctcttcaat gctgccctgg ctgcagcagg aaagatgggc    2640
   cccaggggag ccaagggcgg caagctcgcc cacctgagag ccaggctcaa agaactggct    2700
   gcactggagg cagccgcacg acagcagcag ctgctggaac aggccaagag ctcgcaagaa    2760
   gccgggggag aggagggctc tggagccaca gaccaacctg caccccagga gccgtcagag    2820
   cccaaggagc ccccggagcc cccagccccg agttctccac cgccagcctc agcaaagccc    2880
   gagggaagca cggaggaggc cgcagggccg ccggagcctt cagtgaggat ccgtgtgagt    2940
   ccaggccctg atcccgggga acagacacta tcggtggaga tgctggaaga gaagaaggag    3000
   gaggtggagt gaggtgcatt cagcaccctg atcccatccc taggtgcgga ccagaccttt    3060
   gaaccacctg cctcaccaag catcttagcc taaaggcgcc ttctcaccgg cttatagaaa    3120
   ctctcaaatt tcatcaagta gtggtggtga cctcgagctc ctaacattcc cggagcaccc    3180
   tgctcccctc tcacagttgc tattttctag aagaaaaccg ccttgcactt taaaaa        3236
<210> 3
   <211> 2351
   <212> DNA
   <213> Rattus norvegicus
<400> 3
   cgctggagcc cgagggcagc ctcttccagg aagccaggca ctttcccaag ccacctataa      60
   tcggagaatc actggatagt ctacagacta gaaccacact acggaaacat gagccaggac     120
   ggcaaagtga agaccacaga gtccacgccc ccagccccaa ccaaagccag gaagtggctg     180
   ccagtcctag acccatctgg ggattactac tactggtggc tgaacacaat ggtcttccca     240
   atcatgtaca acctcatcat cgttgtatgc agggcctgct ttcctgactt gcagcacagt    300
   tacctggtgg cctggttcgt gctggactac acgagtgacc tgctgtacct actagatatt    360
   ggggtacgct tccacacagg attcctagag cagggcatcc tggtggtaga caaaggcatg    420
   atcgccagtc gctacgtccg cacctggagc ttcctgttgg acctggcgtc cctggtcccc    480
   acagatgcgg cctatgtgca gctgggcccc cacatcccta cactccggct aaaccgcttt    540
   ctccgagtgc cccgcctctt cgaggctttt gatcgtacag agacccgcac ggcttaccca    600
   aatgccttcc gcatagccaa gctgatgctt tacatttttg ttgtcatcca ttggaacagt    660
   tgcttatact tcgccctgtc caggtacctg ggctttggac gggatgcgtg ggtataccca    720
   gaccctgcgc aacctggctt tgagcgcttg cggcgccagt atctctacag cttctacttc    780
   tccactctga tcctgaccac agtgggtgac acgccgctgc cagaccgaga ggaagagtac    840
   ctcttcatgg tgggtgactt cctgctggcc gtcatgggtt tcgccaccat catgggtagc    900
   atgagctctg tcatttacaa catgaacact gcagatgcgg ccttctaccc agaccatgcg    960
   ctggtaaaga agtacatgaa gctgcagcat gtcaacaaga ggctggagcg gcgagttatt   1020
   gactggtacc agcatcttca gatcaacaag aagatgacca acgaggtagc catcttgcag   1080
   cacctgcctg agcggctgcg ggcggaggtt gctgtgtccg tgcacctgtc taccctgagc   1140
   cgagtacaga tcttccagaa ctgtgaagcc agcctgctgg aagagctggt gctgaagcta   1200
   cagccccaga cctactcgcc aggcgaatat gtgtgccgca aaggggacat tggccgagag   1260
   atgtacatca tccgtgaggg ccagctggct gtggtggccg atgatggtgt cacacagtat   1320
   gctgtgcttg gtgcagggct ctactttggg gagatcagta tcatcaacat caaagggaac   1380
   atgtctggaa accgacgaac agccaacatc aagagcctag gttattcaga cctgttctgc   1440
   ctcagcaagg aggatctgcg ggaggtactg agtgagtacc cacaggccca ggcggtcatg   1500
   gaggagaagg gccgagaaat cttgctcaaa atgaataagt tggatgtgaa tgctgaggca   1560
   gctgagatcg ccctccagga ggccacagag tctcggctca aaggcctcga ccagcagctt   1620
   gatgatctgc agaccaagtt tgctcgccta ctggctgagc tggagtccag tgcactgaag   1680
   atagcttacc gcatcgaaag gctggagtgg cagactcgag agtggccaat gccagaggac   1740
   atgggtgagg ctgatgatga ggctgagcct ggagaaggga cgtccaagga tggagaggga   1800
   aaggctggcc aggcgggacc ctcaggcata gaatgacccc gtcctgaccc taggactccc   1860
   agctcaaata aatccagagt ggtgggaaag tctgcctgca ggaactgtca tcctctttgc   1920
   taggttacag aacgtaggta aattggtcta tagatgccta gctagggatg tgggtcacag   1980
   catccattag tcccatactc accagcaaat gtacacacac acacacacac aaacatgcgc   2040
   acgcacacgc tcaagactga gttctacata atgttcctgt cttctgcaca catgtgcacc   2100
   atgatcacag agaacaattg aatgcactgg gactcttgag gtaagcttta cacatcttaa   2160
   aggaggtctg ctggtttagg gcaaggacag tgttggagct acagggaaag tagacagata   2220
   caattcctga actctaggtc accacttcag ctgcttcagg gtatggcccg acttcaggtc   2280
   tgagctggga caggcggtat tttgaaatga acctgatgtg tgatttatta ataaaatata   2340
   aggttcataa c                                                        2351
<210> 4
   <211> 1992
   <212> DNA
   <213> mutated CNG2
<400> 4
   atgacagaaa aagccaatgg cgtgaagagc tccccagcca ataaccacaa ccaccatgcc 60
   cctcctgcca tcaaggccag tggcaaagat gaccacaggg ccagcagccg gccacagtct 120
   gctgctgctg atgacacctc ctcagagcta cagcaactgg cagagatgga tgccccccag 180
   cagaggaggg gtggcttccg caggattgcc cgcctggtgg gggtcctcag agagtgggct 240
   tacaggaact tccgtgagga ggagcctaga cctgactcat tccttgagcg tttccggggg 300
   cctgagctcc acaccgtgac aacacaacaa ggagacggca aaggcgacaa ggacggcgag 360
   ggcaagggca ccaagaagaa gtttgaactc tttgtcttgg acccagccgg ggactggtac 420
   taccgctggc tttttctcat tgccttgccc gtcctctaca actggtgcct attggtggcc 480
   agagcctgct tcagtgacct gcagaaaggc tactacatag tgtggctggt gctggattac 540
   gtctcagatg tggtctacat cgcagacctc ttcatccgac tgcgcacagg tttcttggag 600
   caggggctac tggtgaaaga caccaagaag ttgcgggaca actacatcca caccatgcag 660
   tttaagctgg atgtggcctc catcatccct acagacctga tctattttgc tgtggggatc 720
   cataaccctg aggtgcgctt caaccgcctg ctacactttg cccgcatgtt tgagttcttt 780
   gaccgcactg agacacgcac cagctacccc aacatcttcc gaataagcaa cctgatcctc 840
   tacatcttga tcatcattca ctggaatgcc tgcatctact atgccatctc caagtccatc 900
   ggctttgggg tagacacctg ggtttacccc aacatcactg accctgagta tggctacctg 960
   tctagggagt acatctattg cctttactgg tctacactga ccctcaccac cattggggag 1020
   acaccacccc ctgtaaagga tgaggagtac ctgtttgtca tctttgactt cctgattggt 1080
   gtcctcatct ttgccaccat cgtgggaaat gtgggctcca tgatctccaa catgaatgcc 1140
   acccgggctg agttccaggc caagattgat gctgtcaaac attatatgca gttccgaaag 1200
   gtcagcaagg agatggaagc caaggtcatt aggtggtttg actacttgtg gaccaataag 1260
   aagagtgtag atgagcgaga agtcctcaaa aacctgccag caaagctcag ggctgagata 1320
   gccatcaacg tccacctgtc cacactcaag aaagtgcgca tctttcagga ctgtgaggct 1380
   ggcctgctgg tggaactggt attaaagctc cggcctcagg tctttagccc tggggactac 1440
   atttgccgca agggggatat tgggaaggag atgtacataa tcaaggaggg aaaattggca 1500
   gtggtggctg atgacggtgt cactcagtat gccctgctct cggctgggag ttgctttgga 1560
   gagatcagta tccttaatat taagggcagc aaaatgggca atcggcgcgc agccaacatc 1620
   cgcagtcttg gctactctga tctgttctgc ttgtccaagg atgatcttat ggaagctgtg 1680
   actgagtacc ctgatgccaa gagggtcttg gaggagagag gccgggagat tctgatgaag 1740
   gagggcttgt tggatgagaa tgaggtggca gccagcatgg aggtagatgt gcaggaaaag 1800
   ctagaacagc tggagaccaa catggacacc ttgtacactc gttttgcccg cctgctggcc 1860
   gagtacacgg gagcccagca gaagctcaag cagcgcatca cagttttgga aacgaagatg 1920
   aagcagaata atgaggatga ctccctgtca gatgggatga acagcccaga gccacctgcc 1980
   gagaagccat aa 1992
<210> 5
   <211> 2641
   <212> DNA
   <213> Bos taurus
<400> 5
   gtcgtcatgg cggaggatgc tgtgcgaggt ggatggcagc cttctcccca ggaccctcca    60
   ggtccgagtc tgtggaggcc tcagcaactg gagtatgtgg acactgccat tcagcagcca   120
   ccttggggtg gaatgtgaca cagcaagaag atggccaaga ttagcaccca atactcccac   180
   cccaccagga cacacccctc ggtcaggacc atggacagag atctggattg cattgaaaac   240
   ggtctcagca ggacccactt gccatgcgag gagacatcgt cagaactgca ggaaggcatt   300
   gccatggaga ctagaggact ggctgaatcc aggcaaagct ccttcaccag ccagggtccc   360
   accaggttgt cacgcctcat catctcgctc cgtgcctgga gcgccaggca cttacaccag   420
   gaggaccaga ggcccgactc tttcctggag cgtttccgcg gagctgagct ccaagaggtg   480
   tctagccgag aaagccatgt ccagttcaat gtgggcagcc aggagccacc agacagaggg   540
   agaagtgcct ggcccctggc cagaaacaac accaacacct gcaacaactc ggagaaggat   600
   gacaaggcga aaaaggagga gaaagagaaa aaggaagaga aaaaggagaa ccccaagaaa   660
   gaggagaaga agaaggacag cgttgtgatg gacccttcca gcaacatgta ctaccactgg   720
   ctgactgtca tcgccgtgcc tgtcttctac aactggtgtc tgctcgtgtg cagggcctgt   780
   ttcgatgagc tccagtccga gcacctgatg ctttggctgg tcctggacta ctcagcagac   840
   atcctctatg gcatggatat gctggtccga gcccggacag gcttcctgga gcaaggcctg   900
   atggtcatgg acgccagccg gctgtggaag cactacacac agaccttgca cttcaagctg   960
   gacgtgttgt ccctggtgcc cacagacctg gcttatttta agctgggcat gaactaccca  1020
   gaactgaggt tcaaccgcct cctgaagttg gcccggctct tcgaattctt tgaccgcacg  1080
   gagacaagga ccaactaccc caacatgttc aggatcggga acttggtctt gtacatcctc  1140
   atcatcatcc actggaatgc ctgcatctac tttgccattt ccaagttcat tggtttcggg  1200
   accgactcct gggtctaccc aaacgtctcc aacccagagt atgggcgcct ctccagaaag  1260
   tacatttaca gtctctactg gtccaccttg accctgacca ccattgggga gaccccgccc  1320
   cccgtaaaag acgaggaata tctctttgtg gtcatcgact tcctggtggg cgtcctgatt  1380
   tttgccacca tcgtgggcaa cgtgggctcc atgatctcaa acatgaatgc ttcacgggcc  1440
   gagttccagg ccaagatcga ttccatcaag cagtacatgc agttccgcaa ggtgaccaag  1500
   gacttggaga cacgggtgat ccgctggttc gactacctgt gggccaataa gaagacagtg  1560
   gatgagaagg aggtgctcaa gagcctcccc gacaagctga aggccgagat cgccatcaac  1620
   gtgcacctgg acaccctgag gaaggtccga atcttccagg actgcgaggc ggggctgctg  1680
   gtggagctgg tgctgaagct gcggccggca gtgttcagcc ccggggacta catctgcaag  1740
   aagggggaca tcgggaggga gatgtacatt atcaaggagg gcaagctggc cgtggtggcc  1800
   gaggacggca tcacccagtt cgtggtcctc ggcgacggga gttatttcgg ggagatcagc  1860
   atcttgaaca tcaaggggag caagtccggg aaccgccgca cggccaacat caggagcatc  1920
   ggctactcgg acctgttctg cctctccaag gacgacctga tggaggcgct caccgagtac  1980
   cccgaggcca agaaggcgct ggaggagaaa gggcggcaga tcctcatgaa ggacaacctg  2040
   atcgacgagg agctggccaa ggccggggca gaccccaagg acatcgagga gaaggtggag  2100
   cacctcgaga cctccctgga ctccctgcag accaggtttg cgcggctcct ggctgagtac  2160
   aacgccaccc agatgaaggt gaagcagcgg ctcagccagt tggaaagcca ggtgaagatg  2220
   ggcctcccgc ctgatggcga tgctccgcaa actgaggcca gtcagccctg aagacacagg  2280
   tgccctctcc tgcctccccg gggcggcggt cagtgcgacg ctgcgccgca cggggctcgg  2340
   ccgggaccga attctagctt tccccaccct ctgtgcgctg cgtggccttg ggagagagcc  2400
   ttggtttccc tcatctagat aacaggactc tttatgtctg tcccagttaa gtgacaggtt  2460
   gctgtgagct ccacaagaaa cgcttcgtga ggcagggttt tgttaagtgt gagatgtttc  2520
   taggccaaga gtataaaaat gtgagcacag aagttgtttt tttttttttt ttttaatccg  2580
   tggggaatat ttagactcct gaacttcatt ttttttgtaa atgggaggtt atttacttac  2640
   c                                                                  2641

## Beschreibung

Die Erfindung betrifft Verfahren zur quantitativen optischen Analyse der intrazellulären Konzentration von zyklischem Guanosin-3',5'-monophosphat (cGMP) bzw. zyklischem Adenosin-3',5'-monophosphat (cAMP). Diese Verfahren können zur Identifizierung und Evaluierung der pharmakologischen Eigenschaften von Testsubstanzen dienen, die die Aktivität von Rezeptoren bzw. Enzymen beeinflussen, die am Aufbau bzw. Abbau der zyklischen Nukleotide cGMP und cAMP beteiligt sind. Damit eignet sich das Verfahren beispielsweise zum Auffinden von Modulatoren der löslichen und membrangebundenen Guanylatzyklasen, Phosphodiesterasen (PDE), NOsynthesen und G-Protein gekoppelten Rezeptoren (GPCR).

Die Erfindung beschreibt ein Verfahren bei dem die intrazellulären Konzentrationen von cGMP bzw. cAMP mit Hilfe rekombinanter Zelllinien gemessen wird. Diese Zelllinien exprimieren rekombinant eine Kombination bestimmter durch zyklische Nukleotide aktivierte Ionenkanäle (CNG-Kanäle), ein Calcium-sensitives Photoprotein, sowie einen Rezeptor bzw. ein Enzym, für das Modulatoren gesucht werden sollen. Das Verfahren eignet sich zur Automatisierung und zum Auffinden von Modulatoren mit hohem bzw. sehr hohem Durchsatz ("High-Throughput Screening" (HTS) und "ultra High-Throughput Screening" (uHTS)).

Gegenwärtige Verfahren zur Messung der intrazellulären Konzentration von cGMP bzw. cAMP haben den Nachteil, dass sie wegen der Verwendung von Radioaktivität sehr teuer, nicht oder nur teilweise zu automatisieren und sehr aufwendig sind.

Herkömmliche kommerzielle Systeme zur Bestimmung von cGMP und cAMP sind radioaktive Techniken wie der "Radioimmuno-Assay" (RIA; z.B. von IBL, Hamburg), der "Szintillation-Proximity-Assay" (SPA; Amersham Bioscience, UK) und der nichtradioaktive "enzyme-linked immunosorbent assay" (ELISA; z.B. von Amersham Bioscience, UK).

Eine Messung der intrazellulären Konzentration von cGMP und cAMP an lebenden Zellen "insitu" ist mit den oben beschriebenen Verfahren nicht möglich. Die Zellen müssen für die Messungen aufgeschlossen, und die zyklischen Nukleotide dann anschließend extrahiert werden.

Ein weiteres Verfahren zur Messung von intrazellulärem cAMP ist die Messung mit Hilfe von induzierbaren Promotorsystemen ("cAMP-response element", CRE) und dem Enzym Luziferase (Goetz et.al., J. Biomol. Screen. (2000) 5 377-384). Der Nachteil des Verfahrens ist allerdings, dass zur Bildung des Signals sowohl Transkription und Translation ablaufen müssen, dies bedingt eine Dauer der Messung über einen Zeitraum von Stunden. Diese Methode ist somit zur schnellen Messung der aktuellen Konzentrationen von cAMP in Zellen nicht geeignet.

Der Erfindung liegt die Aufgabe zu Grunde, ein verbessertes, nichtradioaktives Verfahren zur Messung der intrazellulären Konzentrationen von cGMP bzw. cAMP zu finden. Dabei sollen die Messungen sehr schnell und auch mit hoher Sensitivität durchgeführt werden können. Das Verfahren sollte automatisierbar und geeignet für ein Screening mit hohem Proben- bzw. Testverbindungsdurchsatz (HTS und uHTS) sein.

Testverbindungen im Sinne der Erfindung sind bevorzugt kleinmolekulare chemische Verbindungen mit einem Molekulargewicht von 100 bis 500 oder 100 bis 1000 oder aber auch darüber hinaus. Diese können einzeln oder aber auch als Gemisch in den erfindungsgemäßen Verfahren eingesetzt werden. Zu Testverbindungen im Sinne der Erfindung zählen auch Antikörper, Naturstoffe, Naturstoffextrakte, Peptide, Proteine sowie Nukleinsäuren. Diese Aufzählung ist nur beispielhaft und nicht als abschließend zu werten.

Um ein Screeningsystem für Enzyme und Rezeptoren zu etablieren, die den intrazellulären Spiegel von cAMP bzw. cGMP beeinflussen, werden verschiedene, durch zyklische Nukleotide aktivierte Ionenkanäle (CNG-Kanäle), allein oder in verschiedenen Kombinationen in Zellen transfiziert, die zusätzlich rekombinant ein Calcium-sensitives Photoprotein exprimieren. Anschließend werden diese Zellen mit verschiedenen Enzymen oder Rezeptoren transfiziert, um die Eignung der Zellen für das Screening mit hohem Durchsatz zu prüfen. Hierbei wird zum Beispiel die lösliche Guanylatzyklase verwendet, da sie nach Stimulation zur Bildung von intrazellulärem cGMP in der Lage ist. Als Rezeptor kann zum Beispiel der ß-adrenerge Rezeptor verwendet werden, der nach Aktivierung einen Anstieg der intrazellulären cAMP-Konzentration induziert.

CNG-Kanäle sind integrale Membranproteine, die durch die zyklischen Nukleotide cGMP und cAMP geöffnet werden. Es handelt sich bei dieser Familie von Ionenkanälen um nichtselektive Kationenkanäle, die permeabel sind für Natrium-, Kalium- und Calciumionen. Die funktionellen Kanäle werden gebildet aus vier identischen oder auch verschiedenen Untereinheiten (Homo- bzw. Heterotetramere). Über die Kombination verschiedener Untereinheiten lassen sich die Sensitivitäten für cGMP und cAMP beeinflussen (Biel et.al. TCM (1996) 6, 274-280; Kaupp und Seifert, Physiol. Rev. (2002) 82, 769-824). Kloniert und publiziert sind die Kanaluntereinheiten CNG1-CNG6 , die nach neuerer Nonnenklatur auch als CNGA1-CNGB3 bezeichnet werden (Bradley et.al., Science (2001) 294, 2095-2096). Es existieren auch Spleissvarianten wie zum Beispiel CNG4.3 (Saute et.al., PNAS (1998) 95, 4696-4701). DE 101 388 76 A offenbart die Verwendung der Zelllienien die CNG-kanäle enthalten zur Bestimmung der intrazellulären Ca²⁺- konzentration.

Der Anstieg der intrazellulären Konzentration von cGMP bzw. cAMP führt zur Öffnung der CNG-Kanäle und dadurch zu einem Einstrom von Calciumionen. Das einströmende Calcium kann nun mit Hilfe von Calcium-sensitiven Fluoreszenz-Indikatoren, wie zum Beispiel FURA, Fluo-3 usw., oder mit Hilfe Calcium-sensitiver Photoproteine, wie Aequorin oder Obelin nachgewiesen werden.

Das Photoprotein Aequorin, welches aus der Qualle *Aequorea victoria* stammt, ist ein hochsensitiver Calciumindikator in Zellen. Der Aequorinkomplex besteht aus dem Protein Apoaequorin, molekularem Sauerstoff und dem Luminophor Coelenterazin. Bei Anwesenheit von Calcium (Ca²⁺-Ionen) emittiert dieser Komplex blaues Licht mit einem Emissionsmaximum von 466 nM (Jones et.al., Trends Biotechnol. (1999) 17,477-481).

Ein weiteres Photoprotein ist das Obelin, welches aus verschiedenen Hydrozoen wie z.B. *Obelia longissima* kloniert wurde. Es besteht aus Apoobelin, O₂ und dem Luminophor Coelenterazin. Bei Anwesenheit von Calcium wird ebenfalls blaues Licht emittiert (Illarionov et.al., Methods Enzymol. (2000) 305,223-49).

Guanylatzyklasen sind verantwortlich für die Bildung von cGMP aus GTP. Man unterscheidet lösliche und membrangebundene Guanylatzyklasen. Die lösliche Guanylatzyklase ist ein Heterodimer aus alphal- und betal-Untereinheit. Sie ist der natürliche Rezeptor für das von der endothelialen NO-Synthase gebildete Stickstoffmonoxid (NO) und lässt sich daher auch pharmakologisch durch NO-freisetzende Substanzen, wie zum Beispiel Sie-1, aktivieren. Da das von der Guanylatzyklase gebildete cGMP eine Relaxation von Blutgefässen induziert, handelt es sich bei dem Enzym um ein hochinteressantes pharmakologisches Target (Hobbs AJ., TIPS (1997) 18, 484-491). Die membrangebundenen Guanylatzyklasen besitzen ein Transmembransegment und werden durch verschiedene Agonisten wie ANP, BNP, CNP oder Guanylin aktiviert (Wedel und Garbers, Annual Rev. Physiol. (2001) 63, 215-233).

Eine weitere pharmakologisch interessante Proteinfamilie sind die sogenannten G-Protein gekoppelten Rezeptoren (GPCR). Hierbei handelt es sich um integrale Membranproteine, die die Wirkung eines extrazellulären Hormons in das Innere einer Zelle weiterleiten können. Über die Kopplung dieser Rezeptoren an verschiedene G-Proteine werden die intrazellulären Spiegel von cAMP oder Calcium beeinflusst. Die Aktivierung sogenannter Gq-gekoppelter Rezeptoren führt zu einer Freisetzung von Calcium aus internen Speichern und damit zu einer Erhöhung der zytoplasmatischen Calciumkonzentration. Die Aktivierung sogenannter Gs-gekoppelter Rezeptoren führt dabei zu einem Anstieg in der cAMP-Konzentration, die Aktivierung der sogenannten Gi-gekoppelten Rezeptoren führt dagegen zu einer Senkung des intrazellulären cAMP-Gehalts. (Gurrath M., Curr. Med. Chem. (2001) 8, 1605-1548) Ein Beispiel für Gs-gekoppelte Rezeptoren bilden die sogenannten ß-adrenergen Rezeptoren, die sich mit Hilfe von Agonisten wie Isoprenalin stimulieren lassen (Dzimiri N., Pharmakol. Rev. (1999) 51, 465-501).

Die durch Guanylatzyklasen und GPCRs gebildeten zyklischen Nukleotide cGMP bzw. cAMP werden durch sogenannte Phosphodiesterasen (PDEs) zu GMP bzw. AMP hydrolysiert. Die Phosphodiesterasen bilden eine Enzymfamilie, deren Mitglieder sich bezüglich der Substratspezifität, Regulation und Expressionsmuster im Körper deutlich unterscheiden (Francis et.al., Prog. Nucleic Acid Res. Mol. Biol. (2001) 65, 1-52). PDEs spielen bei der Kontrolle der intrazellulären cAMP- und cGMP-Spiegel eine entscheidende Rolle und sind daher ebenfalls wichtige Targets für die pharmakologische Intervention.

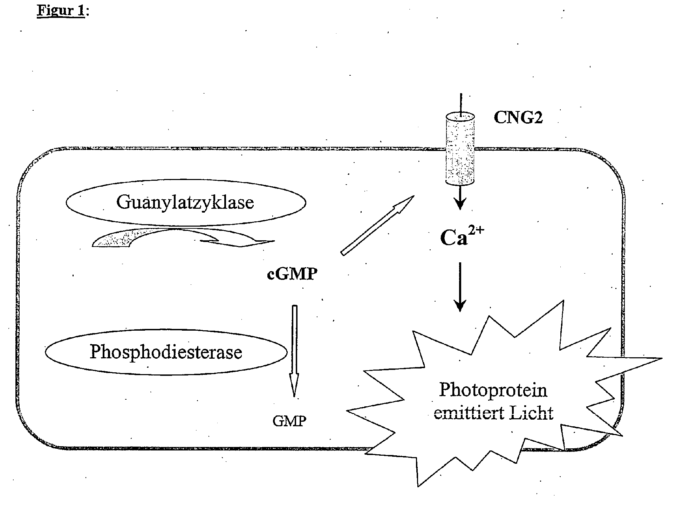

Überraschenderweise zeigte sich, dass die Kombination des CNG3-Kanals (SEQ ID NO: 5, Acc. No. X 89600) oder insbesondere des CNG2-Kanals (Seq ID Nr.1, Acc. No. X55010) mit einem Photoprotein besonders dazu geeignet ist, um Veränderungen der intrazellulären cGMP-Konzentrationen zu detektieren (Figur 1).

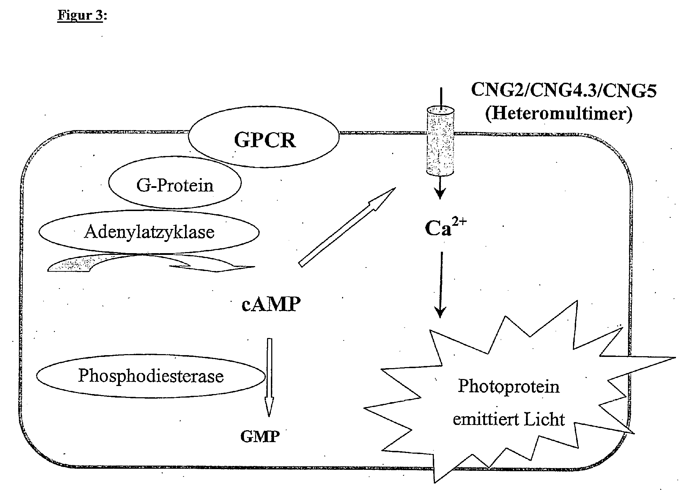

Ebenso überraschend erwies sich eine Kombination aus CNG2/CNG4.3/CNG5 (Seq ID Nr.1/Seq ID Nr.2, Acc. No. AJ000515/Seq ID Nr.3, Acc. No. U12623) mit einem Photoprotein als sehr gut geeignet, um Veränderungen der intrazellulären cAMP-Konzentration zu detektieren (Figur 3). Als besonders gut für die cAMP-Messung geeignet erwies sich die Kombination von CNG2(T537A)/CNG4.3/CNG5 (Seq ID Nr.4/Seq ID Nr.2/Seq ID Nr.3) mit einem Photoprotein. Bei der CNG2-Mutante CNG2(T537A) (Seq ID Nr.4) ist die Aminosäure Threonin-537 gegen Alanin ausgetauscht (Altenhofen et.al., PNAS (1991) 88, 9868-9872).

Zur Herstellung der rekombinanten Zelllinien können gängige Vektoren wie pcDNA1.1 Amp oder pcDNA3 (Invitrogen Life Technologies) verwendet werden. Für die Transfektionen der entsprechenden Plasmidkonstrukte können gängige Transfektionsreagentien wie beispielsweise Lipofectamin (Invitrogen Life Technologies) verwendet werden.

Die Zellen werden für die Messungen auf 384-Loch Mikrotiterplatten (MTP) mit 1.500 Zellen pro Loch, auf 1536-Loch MTP mit 250 Zellen pro Loch ausgesäht. Nach 1-2 Tagen Wachstum bei 37°C/5% CO₂ wird das Zellkulturmedium (DMEM/F12 mit 10% fötalem Kälberserum) entfernt und die Zellen für 3h mit Coelenterazin (0,8 µg/ml) in Calcium-freier Tyrode bei 37°C/5% CO₂ beladen. Anschließend werden Testsubstanzen und entsprechende Kontollsubstanzen (z.B. SIN-1 oder Isoprenalin) in Calcium-freier Tyrode zugegeben und für 5-10 min auf den Zellen inkubiert. Anschließend erfolgt die eigentliche Messung in einer lichtdichten Box unter Zugabe von Calcium-haltiger Tyrode über einen Kamm (Calcium-Endkonzentration: 3 mM) mit Hilfe einer "Charge-compled device"-Kamera.

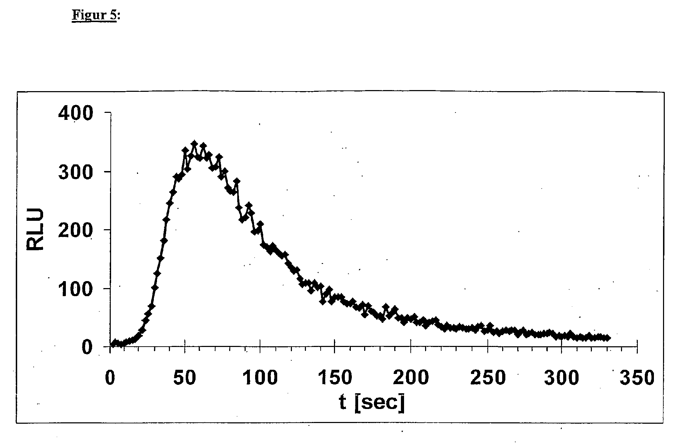

Zur direkten Messung von Substanzeffekten werden die Zellen mit Coelenterazin in Calcium-haltiger Tyrode beladen. Die Zugabe der Substanzen erfolgt dann innerhalb der lichtdichten Box und die Messung beginnt unmittelbar mit der Substanzzugabe (Figur 5).

Die Vorteile dieses Verfahrens liegen in der hohen Sensitivität der Messung, den geringen Kosten pro Messpunkt und der Eignung für das HTS bzw. uHTS begründet. Das Verfahren besitzt ein ausgezeichnetes Signal-Rauschverhältnis, es lassen sich Stimulationsfaktoren von 50-150-fach erzielen. Außerdem können Prüfsubstanzen für sehr kurze Zeiträume appliziert werden, da bereits nach wenigen Sekunden ein Signal zu beobachten ist (Figur 5). Dies hat den Vorteil, dass unspezifische Effekte von Prüfsubstanzen (z.B. durch Zytotoxizität) auf die verwendeten Zellen minimiert werden. Außerdem eignet sich das Verfahren zur Charakterisierung von sogenannten "Orphanrezeptoren" (Rezeptoren deren natürlicher Ligand nicht bekannt ist), da sowohl Änderungen im cAMP-Spiegel (Gs- und Gi-Kopplung) als auch Änderungen der intrazellulären Calciumkonzentration (Gq-Kopplung) mittels Lumineszenz- oder Fluoreszenzmessung beobachtet werden können.

Die Erfindung betrifft Verfahren zur Bestimmung der intrazellulären Konzentration des zyklischen Nukleotides cAMP, dadurch gekennzeichnet, dass
i) eine Zelle hergestellt und verwendet wird, welche die durch zyklische Nukleotide aktivierbaren Ionenkanäle CNG2(T537A)/CNG4.3/CNG5 zusammen mit einem Ca²⁺-sensitiven Photoprotein exprimiert
   und
ii) die intrazelluläre Konzentration zyklischer Nukleotide mittels des Lumineszens-Signals des Photoproteins bestimmt wird.

Gegenstand der Erfindung sind Verfahren wie oben beschrieben, wobei das Photoprotein Aequorin ist.

Die Erfindung betrifft auch wie oben beschriebene Verfahren, wobei das Photoprotein Obelin ist.

Erfindungsgemäß ist eine Methode zum Screenen von Testverbindungen zur Identifizierung von Rezeptor-Liganden, wobei eines der oben beschriebenen Verfahren verwendet wird, in dem die verwendete Zelle den Rezeptor exprimiert und ein intrazelluläres Messenger System besitzt, welches es einer Rezeptor-Liganden Bindungen erlaubt, eine messbare Modulation des Ionenflusses durch den Ionenkanal auszulösen, diese Zelle mit Testsubstanzen inkubiert wird und solche Testsubstanzen ausgewählt werden, welche die Lumineszens modulieren.

Die Erfindung betrifft auch eine solche Methode, wobei es sich bei dem Rezeptor um einen G-Protein gekoppelten Rezeptor handelt.

Erfindungsgemäß ist auch eine dementsprechende Methode, wobei es sich bei dem G-Protein gekoppelten Rezeptor um einen orphanen Rezeptor handelt.

Gegenstand der Erfindung ist desweiteren eine Methode zum Screenen von Testverbindungen zur Identifizierung von Modulatoren von Phosphodiesterasen, wobei eines der oben beschriebenen Verfahren verwendet wird, in dem die verwendete Zelle die Phosphodiesterase exprimiert, diese Zelle mit Testsubstanzen inkubiert wird und solche Testsubstanzen ausgewählt werden, welche die Lumineszens modulieren.

Die Erfindung betrifft auch eine Zelle, welche nach einem der zuvor geschilderten Verfahren hergestellt wurde.

### Beschreibung der Abbildungen

- Figur 1:: Schematische Darstellung des Verfahrens zur Bestimmung der intrazellulären cGMP-Konzentration.
- Figur 2:: Lumineszenzmessung nach Aktivierung der löslichen Guanylatzyklase mit SIN-1. Die Messung der "relativen Lichteinheiten" (RLU = "relative light units") erfolgte über 1 min. Dargestellt ist die bei der angegebenen SIN-1 Konzentration in diesem Zeitraum gemessene Lichtmenge.
- Figur 3:: Schematische Darstellung des Verfahrens zur Bestimmung der intrazellulären cAMP-Konzentration.
- Figur 4:: Lumineszenzmessung nach Aktivierung des β-adrenergen Rezeptors mit Isoprenalin. Die Inkubationszeit mit Isoprenalin beträgt 10 min in Calcium-freier Tyrode. Start der Messung durch Zugabe von Calcium. Die Messung der "relati- ven Lichteinheiten" (RLU = "relative light units") erfolgt in Sekundenintervallen. Dargestellt ist der zeitliche Verlauf des Lumineszenzsignals nach Zugabe von Cal- cium und vorheriger Inkubation mit 0 M (Sterne), 10⁻⁷ M (Vierecke) und 10⁻⁶ M (Dreiecke) Isoprenalin.
- Figur 5:: Kinetik des Lumineszenzsignals nach Aktivierung des β-adrenergen Rezeptors mit Isoprenalin in Calcium-haltiger Tyrode. Start der Messung erfolgt bei Zugabe des Agonisten Isoprenalin (10⁻⁶ M) innerhalb der lichtdichten Box.

### Beispiele

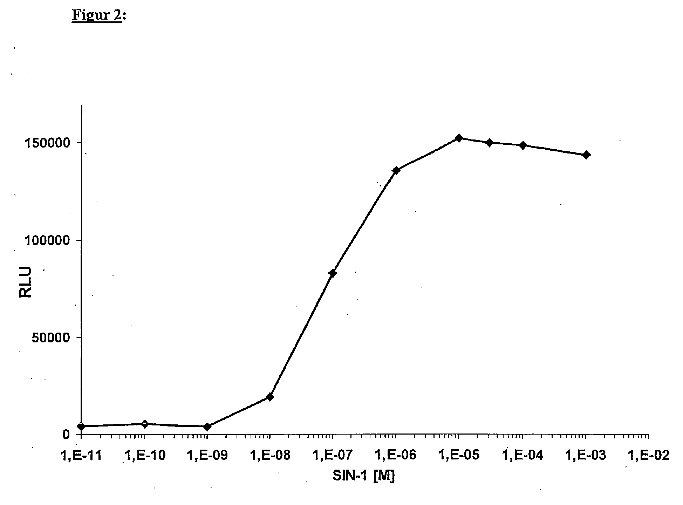

Exprimiert man beispielsweise die lösliche Guanylatzyklase zusätzlich in einer Zelllinie, die als cGMP-Messsystem den CNG2-Kanal (Seq ID Nr.1) zusammen mit einem Calcium-sensitiven Photoprotein exprimiert, so lässt sich mit Hilfe des Guanylatzyklase-Stimulators SIN-1 dosisabhängig die intrazelluläre cGMP-Konzentration erhöhen und ein verstärktes Lumineszenzsignal detektieren (Figur 2).

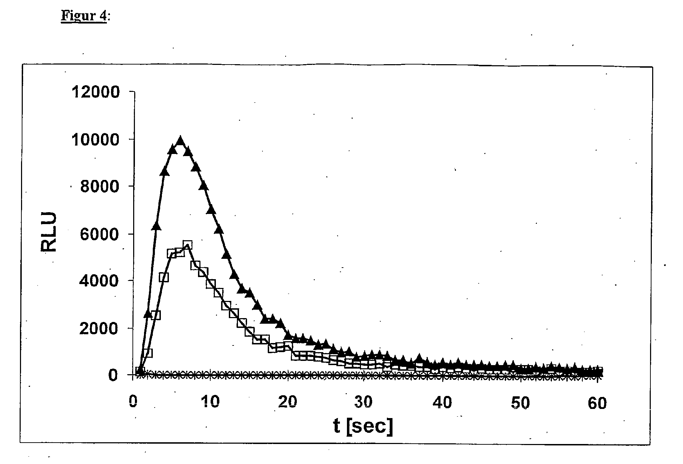

Exprimiert man beispielsweise den β-adrenergen Rezeptor zusätzlich in Zellen, die als cAMP-Messsystem ein Calcium-sensitives Photoprotein zusammen mit den CNG-Kanaluntereinheiten CNG2(T537A)/CNG4.3/CNG5 (Seq ID Nr.4/Seq ID Nr.2/Seq ID Nr.3) exprimieren, so führt die Aktivierung dieses Rezeptors mittels Isoprenalin dosisabhängig zu einer erhöhten cAMP-Konzentration und einem verstärkten Lumineszenzsignal (Figur 4). Werden die Zellen mit Coelenterazin in Calcium-haltiger Tyrode beladen, so kann der Agonist Isoprenalin auch direkt innerhalb der lichtdichten Box zu den Zellen hinzugegeben werden. Man beobachtet dann innerhalb weniger Sekunden ein Lumineszenzsignal (Figur 5).

## Patentansprüche

1. Verfahren zur Bestimmung der intrazellulären Konzentration des zyklischen Nukleotides cAMP, **dadurch gekennzeichnet, dass**
i) eine Zelle hergestellt und verwendet wird, welche die durch zyklische Nukleotide aktivierbaren Ionenkanäle CNG2(T537A)/CNG4.3/CNG5 zusammen mit einem Ca²⁺-sensitiven Photoprotein exprimiert
und
ii) die intrazelluläre Konzentration zyklischer Nukleotide mittels des Lumineszens-Signals des Photoproteins bestimmt wird.

2. Verfahren nach Anspruch 1 , wobei das Photoprotein Aequorin ist.

3. Verfahren nach Anspruch 1, wobei das Photoprotein Obelin ist.

4. Eine Methode zum Screenen von Testverbindungen zur Identifizierung von Rezeptor-Liganden, wobei ein Verfahren nach einem der Ansprüche 1-3 verwendet wird, in dem die verwendete Zelle den Rezeptor exprimiert und ein intrazelluläres Messenger System besitzt, welches es einer Rezeptor-Liganden Bindungen erlaubt, eine messbare Modulation des Ionenflusses durch den Ionenkanal auszulösen, diese Zelle mit Testsubstanzen inkubiert wird und solche Testsubstanzen ausgewählt werden, welche die Lumineszens modulieren.

5. Eine Methode nach Anspruch 4, wobei es sich bei dem Rezeptor um einen G-Protein gekoppelten Rezeptor handelt.

6. Eine Methode nach Anspruch 5, wobei es sich bei dem G-Protein gekoppelten Rezeptor um einen orphanen Rezeptor handelt.

7. Eine Methode zum Screenen von Testverbindungen zur Identifizierung von Modulatoren von Phosphodiesterasen, wobei ein Verfahren nach einem der Ansprüche 1-3 verwendet wird, in dem die verwendete Zelle die Phosphodiesterase exprimiert, diese Zelle mit Testsubstanzen inkubiert wird und solche Testsubstanzen ausgewählt werden, welche die Lumineszens modulieren.

8. Zelle, welche die durch zyklische Nukleotide aktivierbaren Ionenkanäle CNG2(T537A)/CNG4.3/CNG5 sowie ein Ca²⁺-sensitives Photoprotein exprimiert.

## Claims

1. Method for determining the intracellular concentration of the cyclic nucleotide cAMP, **characterized in that**
i) a cell is produced and used, which expresses together with the Ca²⁺-sensitive photoprotein the ion channels activated by the cyclic nucleotides GNG2(T537A)/CNG4.3/CNG5
and
ii) determining the intracellular concentration of cyclic nucleotides by means of the luminescence signal of the photoprotein.

2. The method of claim 1, wherein the photoprotein is aequorin.

3. The method of claim 1, wherein the Photoprotein is obelin.

4. A method of screening test compounds for the identification of receptor ligands, using a method according to anyone of claims 1 to 3, in which the used cell expresses the receptor and possess an intracellular messenger system, which allows a receptor-ligand bond to induce a measurable modulation of the ion flux through the ion channel, this cell is incubated with test substances and such test substances are selected from those ones which modulate the luminescence.

5. A method according to claim 4, whereby the receptor is a G protein-coupled receptor.

6. A method according to claim 5, whereby the G-protein coupled receptor is an orphan receptor.

7. A method for screening test compounds to identify modulators of phosphodiesterases, using a method according to anyone of claims 1 to 3, in which the used cell expresses the phosphodiesterase, this cell is incubated with test substances and such test substances are selected from those ones which modulate the luminescence.

8. Cell, which expresses the ion channels activated by cyclic nucleotides CNG2(T537A)/CNG4,3/CNG5 and a Ca²⁺-sensitive photoprotein.

## Revendications

1. Procédé pour l'évaluation de la concentration intracellulaire du nucléotide cyclique cAMP, **caractérisé en ce que**
i) une cellule est produite et utilisée, qui exprime, par le biais des canaux ioniques activables par des nucléotides cycliques, CNG2(T537A)/CNG4.3/CNG5 avec une photoprotéine sensible au Ca2+ et
ii) la concentration intracellulaire de nucléotides cycliques est évaluée au moyen du signal luminescent de la photoprotéine.

2. Procédé selon la revendication 1, dans lequel la photoprotéine est l'aequorine.

3. Procédé selon la revendication 1, dans lequel la photoprotéine est l'obéline.

4. Méthode pour le criblage de composés à l'essai pour l'identification de ligands de récepteur dans laquelle un procédé selon l'une des revendications 1 à 3 est utilisé, dans lequel la cellule utilisée exprime le récepteur et dispose d'un système de messager intracellulaire qui permet à des composés ligands d'un récepteur de déclencher une modulation mesurable du flux d'ions dans le canal ionique, cette cellule est incubée avec des substances à l'essai et l'on choisit les substances à l'essai qui modulent la luminescence.

5. Méthode selon la revendication 4, dans laquelle le récepteur est un récepteur couplé à la protéine G.

6. Méthode selon la revendication 5, dans laquelle le récepteur couplé à la protéine G est un récepteur orphelin.

7. Méthode pour le criblage de composés à l'essai pour l'identification de modulateurs des phosphodiestérases dans laquelle un procédé selon l'une des revendications 1 à 3 est utilisé, dans lequel la cellule utilisée exprime la phosphodiestérase, cette cellule est incubée avec des substances à l'essai et l'on choisit les substances à l'essai qui modulent la luminescence.

8. Cellule qui exprime, par le biais de canaux ioniques activables par des nucléotides cycliques, CNG2(T537A)/CNG4.3/CNG5 ainsi qu'une photoprotéine sensible au Ca2+.
